(19) Europäisches Patentamt
European Patent Office
Office européen des brevets

(11) Veröffentlichungsnummer: **0 211 434**
**A2**

(12) **EUROPÄISCHE PATENTANMELDUNG**

(21) Anmeldenummer: 86110785.2

(22) Anmeldetag: 04.08.86

(51) Int. Cl.[4]: **A61K 31/54**, A61K 31/545, A61K 9/02, A61K 47/00

(30) Priorität: 05.08.85 US 762248

(43) Veröffentlichungstag der Anmeldung: 25.02.87 Patentblatt 87/09

(84) Benannte Vertragsstaaten: AT BE CH DE FR GB IT LI LU NL SE

(71) Anmelder: F. HOFFMANN-LA ROCHE & CO. Aktiengesellschaft

CH-4002 Basel(CH)

(72) Erfinder: Behl, Charanjit
27 Cottage Place
Nutley N.J. 07110(US)
Erfinder: Unowsky, Joel
37 Fellswood Drive
Livingston N.J. 07039(US)

(74) Vertreter: Lederer, Franz, Dr. et al
Vanderwerth, Lederer & Riederer
Patentanwälte Lucile-Grahn-Strasse 22
D-8000 München 80(DE)

(54) **Rektale Dosierungsformen für b ta-Laktam-Antibiotika.**

(57) Es werden rektale Dosierungsformen enthaltend ein β-Lactam-Antibiotikum als Wirkstoff, ein Gemisch von höheren Fettsäureglyceriden als Vehikel und Chenodesoxycholsäure oder dessen Natriumsalz als Absorptionsverbesserer zur Förderung der Absorption des Antibiotikums im Rektum beschrieben.

EP 0 211 434 A2

Xerox Copy Centre

## Rektale Dosierungsformen für β-Lactam-Antibiotika

Die vorliegende Erfindung betrifft rektale Dosierungsformen, insbesondere Suppositorien, welche ein β-Lactam-Antibiotikum als Wirkstoff, ein Vehikel und einen Absorptionsverbesserer, d.h. eine Verbindung, welche die Absorption des Antibiotikums im Rektum fördert und damit bewirkt, dass therapeutisch aktive Mengen des Antibiotikums ins Blut gelangen, enthalten.

Es ist bekannt, rektale Dosierungsformen, wie Suppositorien, zu verwenden, um oral inaktive β-Lactam-Antibiotika zu verabreichen. Solche Suppositorien enthalten gewöhnlich das Antibiotikum, ein Vehikel, beispielsweise Mischungen von höheren -($C_{12}$-$C_{18}$) Fettsäureglyceriden, wie diejenigen der Witepsol-Klasse (z.B. Witepsol H-15), und eine Substanz zur Förderung der Absorption des Antibiotikums im Rektum. Substanzen, wie Probenecid und Fettsäuren mit einer $C_8$-$C_{14}$-Kette, sind in der Vergangenheit verwendet worden, um die Absorpiton von Antibiotika aus Suppositorien zu fördern. Darüberhinaus ist auch die Verwendung von Mischungen von $C_2$-$C_{12}$-Fettsäuregylceriden als Absorp tionsverbesserer beschrieben worden.

Erfindungsgemäss verwendet man als Absorptionsverbesserer zur Förderung der rektalen Absorption der β-Lactam-Antibiotika die Chenodesoxycholsäure oder dessen Natriumsalz.

Erfindungsgemäss verwendet man als Vehikel bei der Zubereitung der rektalen Dosierungsformen Mischungen von höheren, insbesondere von $C_{12}$-$C_{18}$-Fettsäuregylceriden, vorzugsweise Glyceride mit Fettsäuren, welche eine gerade Anzahl von Kohlenstoffatomen besitzen.

Als geeignete β-Lactam-Antibiotika seien die bekannten Antibiotika erwähnt, welche als zentrales Ringsystem einen β-Lactam-Ring aufweisen. Als Beispiele seien die Verbindungen der allgemeinen Formel

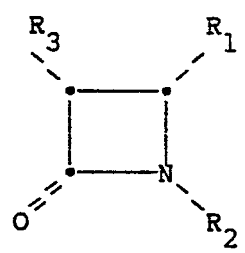

I

worin $R_1$ Wasserstoff oder gegebenenfalls substituier-tes niederes Alkyl und $R_2$ die Gruppe $SO_3^-$ $M^+$ oder $R_1$ und $R_2$ zusammen mit dem β-Lactam-Ring (Azetidinon), an welches sie gebunden sind, eine Verbin-dung der allgemeinen Formel

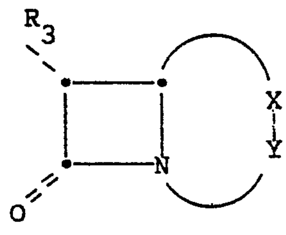

Ia

$M^+$ ein Proton oder ein pharmazeutisch annehmbares Kation, $R_3$ eine Acylamino-oder niedere Hydroxyalkylgruppe, X die Gruppe -S-, -O-, -SO-, $-SO_2-$ oder $-CH_2-$, Y die Gruppe

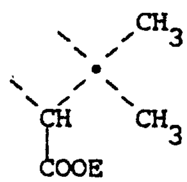

oder

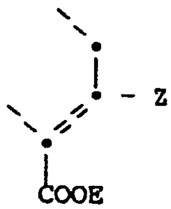

wobei das Kohlenstoffatom, das die Gruppe -COOE trägt, an das Stickstoffatom des β-Lactam-Ringes gebunden ist, Z Wasserstoff, Halogen, niederes Alkoxy oder die Gruppe $-CH_2-T$, T Wasserstoff, niederes Alkanoyloxy, eine Pyridinium-, Carboxamidopyridinium-oder Aminopyridiniumgruppe, Carbamoyloxy, Azido, Cyano, Hydroxy, die Gruppe -S-Phenyl, welche substituiert sein kann, oder die Gruppe -S-het, het einen gegebenenfalls substituierten 5-oder 6-gliedrigen heterocyclischen Ring und E Wasserstoff, eine pharmazeutisch annehmbare Ester-bildende Gruppe oder ein Salzbildendes Kation bedeuten, und die Hydrate dieser Verbindungen erwähnt.

Besonders bevorzugte β-Lactam-Antibiotika sind Cephalosporine, wie Ceftriaxon, Cefamandol und Cefazolin, mono-β-Lactame, wie Carumonam, und Penicilline, wie Piperacillin und Mezlocillin.

In einer ganz besonders bevorzugten Ausführungsform der vorliegenden Erfindung verwendet man Ceftriaxon als β-Lactam-Antibiotikum.

Bevorzugte Vehikel, welche bei der Zubereitung der rektalen Dosierungsformen verwendet werden können, sind die Glyceridgemische, welche vorwiegend aus Triglyceriden von hauptsächlich gesättigten $C_{12}$-$C_{18}$-Fettsäuren, welche vorzugsweise eine gerade Anzahl von Kohlenstoffatomen aufweisen, bestehen. Die am meisten bevorzugten Glyceridgemische sind diejenigen der Witepsol-Klasse, insbesondere das Witepsol H-15. Beispiele derartiger Witepsole sind:

| Witepsol | Steigschmelzpunkt in °C | Erstarrungspunkt in °C | Säurezahl | Verseifungszahl | Jodzahl | Hydroxylzahl | Unverseifbare Bestandteile in % |
|---|---|---|---|---|---|---|---|
| H 5 | 34,0-36,0 | 33,0-35,0 | 0,2 max. | 235-245 | 2 max. | 5 max. | 0,3 max. |
| H 12 | 32,0-33,5 | 29,0-33,0 | 0,2 max. | 240-255 | 3 max. | 15 max. | 0,3 max. |
| H 15 | 33,5-35,5 | 32,5-34,5 | 0,2 max. | 230-240 | 3 max. | 15 max. | 0,3 max. |
| H 175 | 34,5-36,5 | 32,9-34,0 | 0,7 max. | 225-245 | 3 max. | 15 max. | 1,0 max. |
| H 185 | 38,0-39,0 | 34,0-37,0 | 0,2 max. | 220-235 | 3 max. | 15 max. | 0,3 max. |
| H 19 | 33,5-35,5 | 32,0-35,0 | 0,2 max. | 230-240 | 7 max. | 20-30 | 0,3 max. |
| H 32 | 31,0-33,0 | 30,0-32,5 | 0,2 max. | 240-250 | 3 max. | 3 max. | 0,3 max. |
| H 35 | 33,5-35,5 | 32,0-35,0 | 0,2 max. | 240-250 | 3 max. | 3 max. | 0,3 max. |
| H 37 | 36,0-38,0 | 35,0-37,0 | 0,2 max. | 225-245 | 3 max. | 3 max. | 0,3 max. |
| H 39 | 38,0-40,0 | 37,0-39,5 | 0,2 max. | 220-240 | 3 max. | 3 max. | 0,3 max. |
| H 42 | 41,0-43,0 | 40,0-42,5 | 0,2 max. | 220-240 | 3 max. | 3 max. | 0,3 max. |
| W 25 | 33,5-35,5 | 29,0-33,0 | 0,3 max. | 225-240 | 3 max. | 20-30 | 0,3 max. |
| W 31 | 35,0-37,0 | 30,0-33,0 | 0,3 max. | 225-240 | 3 max. | 25-35 | 0,5 max. |
| W 35 | 33,5-35,5 | 27,0-32,0 | 0,3 max. | 225-235 | 3 max. | 40-50 | 0,3 max. |
| W 45 | 33,5-35,5 | 29,0-34,0 | 0,3 max. | 225-235 | 3 max. | 40-50 | 0,3 max. |
| S 55 | 33,5-35,5 | 28,0-33,0 | 1,0 max. | 215-230 | 3 max. | 50-65 | 2,0 max. |
| S 58 | 32,0-33,5 | 27,0-29,0 | 1,0 max. | 215-225 | 7 max. | 60-70 | 2,0 max. |
| E 75 | 37,0-39,0 | 32,0-36,0 | 1,3 max. | 220-230 | 3 max. | 15 max. | 3,0 max. |
| E 76 | 37,0-39,0 | 31,0-35,0 | 0,3 max. | 220-230 | 3 max. | 30-40 | 0,5 max. |
| E 85 | 42,0-44,0 | 37,0-42,0 | 0,3 max. | 220-230 | 3 max. | 15 max. | 0,5 max. |

Die erfindungsgemässen rektalen Dosierungsformen enthalten vorzugsweise etwa 25 mg bis etwa 2000 mg des Wirkstoffes (z.B. Ceftriaxon, Cefamandol, Mezlocillin, Cefazolin, Piperacillin oder Carumonam) insbesondere etwa 50 mg bis etwa 500 mg.

Das Verhältnis Wirkstoff/Vehikel in den erfindungsgemässen rektalen Dosierungsformen liegt zweckmässigerweise zwischen etwa 3:1 und etwa 1:20 und vorzugsweise zwischen etwa 1:1 und 1:3. Am meisten bevorzugt ist ein Verhältnis von etwa 1:2. Das Verhältnis Wirkstoff/Absorptionsverbesserer liegt zweckmässigerweise zwischen etwa 1:2 und etwa 24:1 und vorzugsweise zwischen etwa 2:1 und etwa 8:1. Am meisten bevorzugt wird ein Verhältnis von etwa 4:1.

Auf das $\beta$-Lactam-Antibiotikum bezogen enthalten die rektalen Dosierungsformen vorzugsweise einen Teil $\beta$-Lactam -Antibiotikum, etwa 1-3 Teile des Vehikels und etwa 0,125-0,5 Teile des Absorptionsverbesserers, insbesondere 1 Teil des $\beta$-Lactam-Antibiotikums, etwa 2 Teile des Vehikels und etwa 0,25 Teile des Absorptionsverbesserers. Eine typische rektale Dosierungsform enthält 500 mg des $\beta$-Lactam-Antibiotikums, 1275 mg des Vehikels und 125 mg des Absorptionsverbesserers.

Die erfindungsgemässen pharmazeutischen Kompositionen zur rektalen Verabreichung werden im allgemeinen in Form von rektalen Suppositorien oder von Präparaten, welche erhalten werden, indem man den therapeutischen Wirkstoff, die Chenodesoxycholsäure oder dessen Natriumsalz und das Vehikel und gegebenenfalls andere Bestandteile in einem flüssigen, öligen Träger zu einer fliessfähigen Zusammensetzung (z.B. Suspension, Salbe, Gel, Crème, etc.) dispergiert und diese Zubereitung in rektale Weichgelatinekapseln, Spritzen oder Tuben (z.B. als Klistier) abfüllt.

Die erfindungsgemässen rektalen Dosierungsformen können auch an sich bekannte Hilfsstoffe enthalten, um die gewünschte Konsistenz zu erzielen. Sie können darüberhinaus wasserlösliche Träger, wie Polyäthylenglykol, Polypropylenglykol, Glycerogelatine, Methycellulose oder Carboxymethylcellulose, enthalten. Ebenfalls in Frage kommen Netzmittel, z.B. nicht-ionishce Netzmittel, wie Polyoxyäthylensorbitanfettsäureester, Polyäthylensorbitolfettsäureester, Polyoxyäthylenfettsäureester, Glycerinfettsäureester, beispielsweise Mischungen von Mono-, Di-oder Trigylceriden von Fettsäuren, sowie Polyoxyäthylenester mit höheren Alkoholen, oder anionische Netzmittel, wie Ester von niederen Alkylsulfonsäuren, oder kationische grenzflächenaktive Substanzen. Die rektalen Dosierungsformen können ferner geeignete Emulgier- und Dispergiermittel, Mittel zur Einstellung der Viskosität und Färbemittel enthalten.

Diese Dosierungsformen können erfindungsgemäss hergestellt werden, indem das Vehikel durch Erwärmen (beispielsweise auf Temperaturen oberhalb 45°C, vorzugsweise auf Temperaturen zwischen etwa 45°C und 60°C) schmilzt, den Absorptionsverbesserer und den Wirkstoff und erwünschtenfalls herkömmliche pharmazeutische Hilfsstoffe für rektale Präparate in der erhaltenen Schmelze dispergiert und die erhaltene Dispersion zu rektalen Dosierungsformen wie Suppositorien und Rektalkapseln oder anderen rektalen Verabreichungsformen formuliert.

Die hohe Bioverfügbarkeit der in den erfindungsgemässen rektalen Dosierungsformen enthaltenen Wirkstoffe kann mittels der nachstehend für Ceftriaxon detailliert beschriebenen Methode gezeigt werden, wobei man ein Suppositorium enthaltend die folgenden Bestandteile verwendet:

| | | |
|---|---|---|
| Ceftriaxon-dinatriumsalz-trihydrat [entspricht 502,2 mg (83,7%) Ceftriaxon] | 600 mg | 600 mg |
| Witepsol H-15 | 2675 mg | 1275 mg |
| Chenodesoxycholsäure-Natriumsalz | 125 mg | 125 mg |
| | 3400 mg | 2000 mg |

Die Bioverfügbarkeit von Ceftriaxon wurde under Verwendung eines intravenös verabreichten Standards im bekannten Pavian-Modell ermittelt:

Zur intravenösen Verabreichung wurden die Paviane mit Ketamin-hydrochlorid (5-10 mg/kg), das intramuskulär verabreicht wurde, sediert. Anschliessend entnimmt man eine Anfangsblutprobe und injiziert dann 1 ml einer Ceftriaxon-Lösung in den oberen Teil der Vena saphena an der Beinrückseite.

Für die rektale Verabreichung werden die Paviane während 24 Stunden vor Verabreichung der Wirkstoffe gefastet. Nach Entnahme einer Anfangsblutprobe wird das Suppositorium mittels eines Glasstabes ins Rektum eingeführt. Die Rektalöffnung wird dann während 20 Minuten mit einem Heftpflaster zugeklebt, um das Ausstossen oder Auslaufen des Suppositoriums zu verhindern.

Die Konzentrationen von Ceftriaxon im Plasma wurden wie folgt ermittelt:

Blutproben wurden zur Zeit 0 (vor Verabreichung des Wirkstoffes) und 5, 30, 60, 120, 240, 360, 480, 600, 720 und 1440 Minuten nach Verabreichung des Wirkstoffes entnommen. Die Blutproben wurden mittels einer heparinisierten Spritze aus Venen im Oberschenkelbereich entnommen. Jeweils 1 ml Blut wurde in heparinisierten Zentrifugengläsern während 30 Sekunden zentrifugiert. Das erhaltene Plasma wird entfernt und bis zur Bestimmung der Ceftriaxonkonzentration bei -20°C eingefroren.

Die Plasmaproben wurden mit Acetonitril deproteinisiert, worauf die antibiotische Aktivität in einem mikrobiologischen Versuch auf Agarplatten unter Verwendung von E. coli 1346 als Mikroorganismus bestimmt wird. Die Plasmakonzentrationen wurden dann aufgrund der antibiotischen Aktivität berechnet und als Funktion der Zeit aufgezeichnet, und zwar sowohl für die rektalen als auch für die intravenösen Daten. Die Flächen unter den Kurven (FUK) wurden dann berechnet, und die bereinigten Bioverfügbarkeiten (in %) wurden mittels der folgenden Formel ermittelt:

$$\frac{\text{(FUK rektal)}}{\text{(FUK intravenös)}} \times \frac{\text{(Dosis intravenös)}}{\text{(Dosis rektal)}} \times 100$$

Mit dem obigen Suppositorium, in welchem das Verhältnis Wirkstoff/Absorptionsverbesserer 4:1 beträgt, wurden die folgenden Resultate erhalten:

Durchschnittliche Spitzenkonzentration im Plasma (DSKP) $C_{max}$ = 75,9 ± 19,6 µg/ml; durchschnittliche Bioverfüg barkeit (DB) = 68,5 ± 6,6%

Mittels der obigen Methoden und unter Verwendung ähnlicher Formulierungen wurden für die Verbindungen Carumonam, Piperacillin, Cefamandol, Cefazolin und Mezlocillin die folgenden Resultate erhalten:

Tabelle

| ß-Lactam-Antibiotikum | DSKP $C_{max}$ in µg/ml | DB in % |
|---|---|---|
| Carumonam | 5.6 | 47.0 |
| Piperacillin | 1.2 | 27.0 |
| Cefamandol | 22 | 100.0 |
| Cefazolin | 42 | 55 |
| Mezlocillin | 3 | 43 |

Es ist noch zu erwähnen, dass die niedrigen $C_{max}$-Werte für Carumonam, Mezlocillin und Piperacillin länger anhaltende Werte sind, d.h. dass in diesen Fällen die Bioverfügbarkeit auch bei niedrigen $C_{max}$-Werten erhöht ist.

**Ansprüche**

1. Rektale Dosierungsformen enthaltend eine theapeutisch wirksame Menge eines β-Lactam-Antibiotikums, einen Absorptionsverbesserer zur Förderung der rektalen Absorption des β-Lactam-Antibiotikums und ein Vehikel bestehend aus einer

Mischung von höheren Fettsäureglyceriden, dadurch gekennzeichnet, dass der Absorptionsverbesserer Chenodesoxycholsäure oder dessen Natriumsalz ist.

2. Rektale Dosierungsformen nach Anspruch 1, worin das β-Lactam-Antibiotikum Ceftriaxon, Piperacillin, Carumonam, Cefamandol, Cefazolin oder Mezlocillin ist.

3. Rektale Dosierungsformen nach Anspruch 1 oder 2, worin das β-Lactam-Antibiotikum Ceftriaxon ist.

4. Rektale Dosierungsformen nach einem der Ansprüche 1-3, worin das Vehikel ein Glyceridgemisch aus der Witepsol-Klasse ist.

5. Rektale Dosierungsformen nach Anspruch 4, worin das Witepsol Witepsol H-15 ist.

6. Rektale Dosierungsformen nach einem der Ansprüche 1-5, worin das Verhältnis β-Lactam-Antibiotikum/Vehikel zwischen etwa 3:1 und etwa 1:20 liegt.

7. Rektale Dosierungsformen nach Anspruch 6, worin das Verhältnis β-Lactam-Antibiotikum/Vehikel zwischen etwa 1:1 und etwa 1:3 liegt.

8. Rektale Dosierungsformen nach Anspruch 7, worin das Verhältnis β-Lactam-Antibiotikum/Vehikel etwa 1:2 beträgt.

9. Rektale Dosierungsformen nach einem der Ansprüche 1-8, worin das Verhältnis β-Lactam-Antibiotikum/Chenodesoxycholsäure oder dessen Natriumsalz zwischen etwa 1:2 und etwa 24:1 liegt.

10. Rektale Dosierungsformen gemäss Anspruch 9, worin das Verhältnis β-Lactam-Antibiotikum/Chenodesoxycholsäure oder dessen Natriumsalz zwischen etwa 2:1 und etwa 8:1 liegt.

11. Rektale Dosierungsformen gemäss Anspruch 10, worin das Verhältnis β-Lactam-Antibiotikum/Chenodesoxycholsäure oder dessen Natriumsalz etwa 4:1 beträgt.

12. Rektale Dosierungsformen nach Ansprüchen 7 und 10, worin das Verhältnis β-Lactam-Antibiotikum/Vehikel/Absorptionsverbesserer etwa 1:1-3:0,125-0,5 beträgt.

13. Rektale Dosierungsformen gemäss Ansprüchen 8 und 11, worin das Verhältnis β-Lactam-Antibiotikum/Vehikel/Absorptionsverbesserer etwa 1:2:0,25 beträgt.

14. Rektale Dosierungsformen gemäss einem der Ansprüche 1-5 enthaltend etwa 500 mg des β-Lactam-Antibiotikums, etwa 1275 mg des Vehikels und etwa 125 mg des Absorptionsverbesserers.

15. Rektale Dosierungsformen gemäss einem der Ansprüche 1-14, dadurch gekennzeichnet, dass es sich um Suppositorien, Rektalkapseln, Spritzen oder Tuben handelt.

16. Rektale Dosierungsformen gemäss Anspruch 15, dadurch gekennzeichnet, dass es sich um Suppositorien handelt.

17. Verwendung von Chenodesoxycholsäure oder dessen Natriumsalz in Kombination mit einem Vehikel, wie es in Anspruch 1, 4 oder 5 definiert ist, bei der Herstellung von rektalen Dosierungsformen enthaltend ein β-Lactam-Antibiotikum als Wirkstoff.

18. Verwendung von Chenodesoxycholsäure oder dessen Natriumsalz in Kombination mit einem Vehikel, wie es in Anspruch 1, 4 oder 5 definiert ist, bei der Herstellung von rektalen Dosierungsformen nach einem der Ansprüche 1-16.

19. Verfahren zur Herstellung von rektalen Dosierungsformen enthaltend eine therapeutisch wirksame Menge eines β-Lactam-Antibiotikums, einen Absorptionsverbesserer zur Förderung der rektalen Absorption des β-Lactam-Antibiotikums und ein Vehikel bestehend aus einer Mischung von höheren Fettsäuregylceriden unter Anwendung bekannter technischer Verfahren, dadurch gekennzeichnet, dass man die Chenodesoxycholsäure oder dessen Natriumsalz als Absorptionsverbesserer verwendet.

20. Verfahren gemäss Anspruch 19, dadurch gekennzeichnet, dass man das Vehikel durch Erwärmen schmilzt, den Absorptionsverbesserer und das β-Lactam-Antibiotikum und erwünschtenfalls herkömmliche pharmazeutische Hilfsstoffe für rektale Präparate in der erhaltenen Schmelze dispergiert und die erhaltene Dispersion in eine rektale Dosierungsform überführt.

21. Verfahren gemäss Anspruch 20, dadurch gekennzeichnet, dass die erhaltene Dispersion zu Suppositorien formuliert wird.

22. Verfahren gemäss Anspruch 19, dadurch gekennzeichnet, dass man das β-Lactam-Antibiotikum, das Vehikel und den Absorptionsverbesserer und erwünschtenfalls herkömmliche pharmazeutische Hilfsstoffe für rektale Präparate in einem flüssigen, öligen Träger zu einer fliessfähigen Zubereitung dispergiert und diese Zubereitung in Rektalkapseln, Spritzen oder Tuben abfüllt.

Patentansprüche für den Vertragsstaat : AT

1. Verfahren zur Herstellung von rektalen Dosierungsformen enthaltend eine therapeutisch wirksame Menge eines β-Lactam-Antibiotikums, einen Absorptionsverbesserer zur Förderung der rektalen Absorption des β-Lactam-Antibiotikums und ein Vehikel bestehend aus einer Mischung von höheren Fettsäureglyceriden unter Anwendung bekannter technischer Verfahren, dadurch gekennzeichnet, dass man die Chenodesoxycholsäure oder dessen Natriumsalz als Absorptionsverbesserer verwendet.

2. Verfahren nach Anspruch 1, dadurch gekennzeichnet, dass man das Vehikel durch Erwärmen schmilzt, den Absorptionsverbesserer und das β-Lactam-Antibiotikum und erwünschtenfalls herkömmliche pharmazeutische Hilfsstoffe für rektale Präparate in der erhaltenen Schmelze dispergiert und die erhaltene Dispersion in eine rektale Dosierungsform überführt.

3. Verfahren nach Anspruch 2, dadurch gekennzeichnet, dass die erhaltene Dispersion zu Suppositorien formuliert wird.

4. Verfahren nach Anspruch 1, dadurch gekennzeichnet, dass man das β-Lactam-Antibiotikum, das Vehikel und den Absorptionsverbesserer und erwünschtenfalls herkömmliche pharmazeutische Hilfsstoffe für rektale Präparate in einem flüssigen, öligen Träger zu einer fliessfähigen Zubereitung dispergiert und diese Zubereitung in Rektalkapseln, Spritzen oder Tuben abfüllt.

5. Verfahren nach einem der Ansprüche 1-4, worin das β-Lactam-Antibiotikum Ceftriaxon, Piperacillin, Carumonam, Cefamandol, Cefazolin oder Mezlocillin ist.

6. Verfahren nach einem der Ansprüche 1-5, worin das Verhältnis β-Lactam-Antibiotikum/Vehikel zwischen etwa 3:1 und etwa 1:20 liegt.

7. Verfahren nach einem der Ansprüche 1-6, worin das Vehikel ein Glyceridgemisch aus der Witepsol-Klasse ist.

8. Verfahren nach Anspruch 7, worin das Witepsol Witepsol H-15 ist.

9. Verfahren nach einem der Ansprüche 1-8, worin das Verhältnis β-Lactam-Antibiotikum/Vehikel zwischen etwa 3:1 und etwa 1:20 liegt.

10. Verfahren nach Anspruch 9, worin das Verhältnis β-Lactam-Antibiotikum/Vehikel zwishcen etwa 1:1 und etwa 1:3 liegt.

11. Verfahren nach Anspruch 10, worin das Verhältnis β-Lactam-Antibiotikum/Vehikel etwa 1:2 beträgt.

12. Verfahren nach einen der Ansprüche 1-11, worin das Verhältnis β-Lactam-Antibiotikum/Chenodesoxycholsäure oder dessen Natriumsalz zwischen etwa 1:2 und etwa 24:1 liegt.

13. Verfahren nach Anspruch 12, worin das Verhältnis β-Lactam-Antibiotikum/Chenodesoxycholsäure oder dessen Natriumsalz zwischen etwa 2:1 und etwa 8:1 liegt.

14. Verfahren nach Anspruch 13, worin das Verhältnis β-Lactam-Antibiotikum/Chenodesoxycholsäure oder dessen Natriumsalz etwa 4:1 beträgt.

15. Verfahren nach Ansprüchen 10 und 13, worin das Verhältnis β-Lactam-Antibiotikum/Vehikel/Absorptionsverbesserer etwa 1:1-3:0,125-0,5 beträgt.

16. Verfahren nach Ansprüchen 11 und 14, worin das Verhältnis β-Lactam-Antibiotikum/Vehikel/Absorptionsverbesserer etwa 1:2:0,25 beträgt.

17. Verfahren nach einem der Ansprüche 1-8, dadurch gekennzeichnet, dass man etwa 500 mg des β-Lactam-Antibiotikums, etwa 1275 mg des Vehikels und etwa 125 mg des Absorptionsverbesserers verwendet.

18. Verfahren nach einem der Ansprüche 1-17, dadurch gekennzeichnet, dass man ein Suppositorium, eine Rektalkapsel, eine Spritze oder eine Tube herstellt.

19. Verfahren nach Anspruch 18, dadurch gekennzeichnet, dass man ein Suppositorium herstellt.

20. Verwendung von Chenodesoxycholsäure oder dessen Natriumsalz in Kombination mit einem Vehikel, wie es in Anspruch 1, 7 oder 8 definiert ist, bei der Herstellung von rektalen Dosierungsformen enthaltend ein β-Lactam-Antibiotikum als Wirkstoff.

21. Verwendung von Chenodesoxycholsäure oder dessen Natriumsalz in Kombination mit einem Vehikel, wie es in Anspruch 1, 7 oder 8 definiert ist, bei der Herstellung von rektalen Dosierungsformen nach einem der Ansprüche 1-16.